# EUROPÄISCHE PATENTSCHRIFT

(11) **EP 1 038 867 B1**
(45) Veröffentlichungstag und Bekanntmachung des Hinweises auf die Patenterteilung: **19.05.2004**
(21) Anmeldenummer: 00103942.9
(22) Anmeldetag: 25.02.2000
(51) Int. Cl.: C07D 207/267, C07B 63/00

(54) **Verfahren zur Reinigung von N-substituierten Lactamen, mit sauren makroporösen Kationenaustauschern**
Process for the purification of N-substituted lactames, with acidic macroporous cation exchange resins
Procédé de purification de lactames N-substitués, avec résines acides échangeuses de cation macroporeuses

(30) Priorität: 10.03.1999 DE 19910504
(43) Veröffentlichungstag der Anmeldung: 27.09.2000
(73) Patentinhaber: BASF AKTIENGESELLSCHAFT, 67056 Ludwigshafen (DE)
(72) Erfinder: Henkes, Erhard, Dr., 64683 Einhausen (DE); Iffland, Gabriele, 67071 Ludwigshafen (DE); Ciprian, Jürgen, Dr., 67071 Ludwigshafen (DE)

(56) Entgegenhaltungen:
- WO-A-96/20923
- DE-A- 3 506 473
- DE-A- 3 736 603
- US-A- 3 969 344
- US-A- 5 777 131
- DE DARDEL F. & ARDEN T.V.: "Ion exchangers" ULLMANN'S ENCYCLOPEDIA OF INDUSTRIAL CHEMISTRY (ELVERS B. ET AL., EDS.), FIFTH COMPLETELY REVISED EDITION, DE, VCH VERLAGSGESELLSCHAFT, WEINHEIM, Bd. A14, 1989, Seiten 393-459, XP002140416
- ABRAMS I.M. & MILLAR J.R.: "A history of the origin and development of macroporous ion-exchange resins" REACTIVE & FUNCTIONAL POLYMERS, NL, ELSEVIER SCIENCE PUBLISHERS BV, Bd. 35, Nr. 1-2, 1. Dezember 1997 (1997-12-01), Seiten 7-22, XP004100084 ISSN: 1381-5148

## Beschreibung

Die vorliegende Erfindung betrifft ein Verfahren zur Reduzierung des Amingehaltes von Amin-verunreinigten N-substituierten Lactamen.

N-substituierte Lactame, wie zum Beispiel N-Methyl-2-pyrrolidon (NMP), spielen als polare aprotische Lösungsmittel, als Synthesebausteine und als selektive Extraktionsmittel, z. B. zur Extraktion von Aromaten aus paraffinischen Kohlenwasserstoffen, eine wichtige Rolle. Besonders für Anwendungen in der Elektronikindustrie (z. B. Lithiumionen-Batterien und Photoresiststripper) und der pharmazeutischen Industrie werden hochreine N-substituierte Lactame benötigt.

GB-A-2 088 850 offenbart ein Verfahren zur Behandlung von N-Methyl-2-pyrrolidon (NMP) mit basischen Anionenaustauschern, um saure und/oder korrosive Verunreinigungen im NMP zu entfernen.

US-A-4,831,160 betrifft ein Verfahren zur Reduktion der Konzentration an sauren Komponenten in NMP in mehreren Schritten, wobei der letzte Schritt die Behandlung des NMPs mit basischen Anionenaustauschern umfasst.

DE-A-37 36 603 beschreibt ein Verfahren zur Reindarstellung von N-Vinyl-2-pyrrolidon, das basische Verunreinigungen unbekannter Art enthält, indem man das rohe N-Vinyl-2-pyrrolidon mit einem sauren Kationenaustauscher behandelt. Da in Gegenwart stark saurer Kationenaustauscher eine Polymerisation des N-Vinyl-2-pyrrolidons eintreten kann, sind schwach saure Kationenaustauscher zu bevorzugen (-COOH- Gruppen als Ankergruppen).

US-A-5,777,131 beschreibt ein Verfahren zur Verbesserung der Eigenschaften von N-substituierten Lactamen durch Behandlung der Lactame nach ihrer Herstellung mit Ionenaustauschern, bevorzugt mit stark sauren Kationenaustauschern der Marken Dowex® G23, Dowex® G26, Dowex® HCR-S und Dowex® HGR.
Bei den genannten Kationenaustauschern der Marke Dowex® handelt es sich ausschließlich um mit Sulfonsäuregruppen (-SO₃H) als Ankergruppen funktionalisierte Styrol-Divinylbenzol-Copolymere vom sogenannten Gel-Typ (vergl. Ullmann's Encyclopedia of Industrial Chemistry, Vol. A14, 5^{th} Ed., Seite 451). Die Bezeichnung 'Gel-Typ' bedeutet, dass diese Kationenaustauscher eine natürliche Porosität aufweisen, wobei der Porendurchmesser ca. 1 bis 3 nm beträgt. (Vergl. hierzu: Ullmann's Encyclopedia of Industrial Chemistry, Vol. A14, 5^{th} Ed., Seite 395 und Seite 399f, Kapitel 3.1 (1989)).
Bei der Verbesserung der Eigenschaften der Lactame gemäß US-A-5,777,131 handelt es sich im Fall von NMP um eine Reduktion des Natriumionengehalts von ca. 100 ppb auf weniger als 10 ppb, eine Reduktion des pH-Wertes von ca. 11,5 auf 7 und um eine Reduktion des Amingehaltes von ca. 12 ppm auf weniger als 1 ppm.

EP-A-878 454 offenbart ein Verfahren zur Verringerung des Gehalts an metallischen Kationen, z. B. Alkalimetall- und Erdalkalimetallkationen, in organischen, nahezu wasserfreien Flüssigkeiten, wie z. B. NMP, Isopropylalkohol, Monoethanolamin, Dimethylacetamid, Ethylacetat, Aceton und Sulfolan, durch in Kontakt bringen der organischen Flüssigkeit mit einem sulfonierten Harz auf Grundlage eines Styrol-Divinylbenzol-Copolymers, wobei der Anteil des Divinylbenzols am Gesamtgewicht des Copolymers 50 bis 60 Gew.-% beträgt, wenn man die Sulfonsäuregruppen unberücksichtigt lässt.

US-A 3 969 344 (ACKERMANN J. & RADICI P.) beschreibt die Reinigung von polymerisierbaren Monomeren, unter anderem Caprolactam, mit makroporösen Kationenautauchern in der (Erd-)Alkalimetall Form.

DE-A-35 06 473 (BAYER AG) gibt die Verwendung von makroporösen stark sauren Kationenaustauschern als Katalysatoren bei der Herstellung von Bis-Lactamen an.

WO 96 20923 A (BASF AG) umfasst die Reinigung von (rohem) Caprolactam, mit stark sauren Kationenaustauschern, wobei keine makroporösen Austauscher exemplifiziert wurden.

ABRAMS I.M. & MILLAR J.R.: 'A history of the origin and development of macorporous ion-exchange resins' REACTIVE & FUNCTIONAL POLYMERS, NL, ELSEVIER SCIENCE PUBLISHERS BV, Bd. 35, Nr. 1-2, (1997), Seiten 7-22, ist ein Übersichtsartikel über Ionenaustauscher bzw. makroporöse Ionenaustauscher.

Der vorliegenden Erfindung lag die Aufgabe zugrunde, ein wirtschaftliches, selektives und effizientes Verfahren zur Reduzierung von Aminen, die als Verunreinigung in N-substituierten Lactamen vorliegen, aufzufinden.

Demgemäß wurde ein Verfahren zur Reduzierung von Aminen, die als Verunreinigung in N-substituierten Lactamen vorliegen, gefunden, welches dadurch gekennzeichnet ist, dass man die so verunreinigten N-substituierten Lactame der Formel I
mit einem sauren makroporösen Kationenaustauscher behandelt. 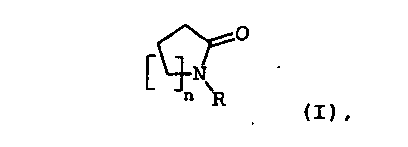 in der R einen linearen oder verzweigten, gesättigten aliphatischen oder cycloaliphatischen Rest darstellt, der ein bis zwei Substituenten, ausgewählt aus Hydroxy und C₁₋₈-Alkoxy, tragen kann, n eine ganze Zahl von 1 bis 4 bedeutet und wobei der heterocyclische Ring des N-substituierten Lactams ein bis zwei Alkylreste tragen kann, mit einem sauren makroporösen Kationenaustauscher behandelt.

Die Vorteile des erfindungsgemäßen Verfahrens liegen darin, dass diese N-substituierten Lactame, die, z.B. bedingt durch das Verfahren ihrer Herstellung, als Verunreinigungen Amine, z.B. primäre Amine der Formel RNH₂, besonders primäre C₁₋₁₂-Alkylamine und C₁₋₄-Monoalkanolamine, wie z.B. Monomethylamin (MMA), Ethylamin, n-Propylamin, iso-Propylamin, n-Butylamin, Cyclopentylamin, Cyclohexylamin, n-Decylamin, n-Dodecylamin, Monoethanolamin, insbesondere MMA, enthalten, mit hoher Selektivität und Effizienz gereinigt werden.

Gleichzeitig werden durch das erfindungsgemäße Verfahren in den N-substituierten Lactamen als Verunreinigungen enthaltene Metallkationen, z.B. Schwermetallionen, wie Fe³⁺, Fe²⁺, Cd²⁺, Co²⁺, Co³⁺, Cr²⁺, Cr³⁺, Cu⁺, Cu²⁺, Mn²⁺, Mn³⁺, Ni²⁺, Pb²⁺, Pb⁴⁺, Sn²⁺, Sn⁴⁺, Zn²⁺ und Ti⁴⁺, und Alkalimetallionen, wie K⁺ und insbesondere Na⁺, und Erdalkalimetallkationen, wie Ca²⁺ und Mg²⁺, abgereichert.

Das erfindungsgemäße Verfahren ermöglicht eine hohe Belastung des makroporösen Kationenaustauschers (Einheit: [BV]; BV = Bettvolumen = Volumen des Ionenaustauschers) und eine hohe spezifische Belastung des makroporösen Kationenaustauschers (Einheit: [BV/h]) bei einer hohen Kapazität des makroporösen Kationenaustauschers (Einheit: [Mol abzureichernde Amin-Verunreinigung / Liter Ionenaustauscher]).

Bevorzugt werden stark saure makroporöse Kationenaustauscher eingesetzt, das heisst solche, die -SO₃⁻ Gruppen (Sulfonsäuregruppen) als Ankergruppen aufweisen.

Besonders bevorzugt handelt es sich um organische makroporöse Kationenaustauscher, insbesondere solche, die ein Styrol-Divinylbenzol-Copolymer als Matrix aufweisen.

Die makroporösen Kationenaustauscher werden im erfindungsgemäßen Verfahren in ihrer sauren Form (in der H-Form) eingesetzt.

Der Porendurchmesser der Makroporen der im erfindungsgemäßen Verfahren eingesetzten Kationenaustauscher beträgt im allgemeinen 10 bis 150 nm und ist damit wesentlich größer als der Porendurchmesser von Kationenaustauschern vom Gel-Typ, die Porendurchmesser von nur ca. 1 bis 3 nm aufweisen.

Bevorzugt weisen die Makroporen der im erfindungsgemäßen Verfahren eingesetzten Kationenaustauscher Porendurchmesser von 20 bis 120 nm, besonders bevorzugt 20 bis 100 nm, ganz besonders bevorzugt 20 bis 40 nm, auf.

Die wesentlich größeren Poren der im erfindungsgemäßen Verfahren verwendeten Kationenaustauscher kommen z.B. dadurch zustande, dass die Herstellung der Styrol-Divinylbenzol-Copolymere in Gegenwart von nicht-polymerisierbaren Verbindungen, wie z.B. Heptan, gesättigten Fettsäuren, C₄₋₁₀-Alkoholen oder Polyalkoholen oder linearem Polystyrol mit geringem Molgewicht, erfolgt. (Vergl. hierzu: Ullmann's Encyclopedia of Industrial Chemistry, Vol. A14, 5^{th} Ed., Seite 395 und Seite 399f, Kapitel 3.1 (1989)).

Die im erfindungsgemäßen Verfahren eingesetzten makroporösen Kationenaustauscher besitzen im allgemeinen eine nutzbare Kapazität von mindestens 0,5 Mol, insbesondere mindestens 0,8 Mol, ganz besonders mindestens 1 Mol, als Verunreinigung vorliegende Amine pro Liter Kationenaustauscher.

Die im erfindunsgemäßen Verfahren verwendeten makroporösen Kationenaustauscher sind nach bekannten Verfahren herstellbar (vergl. Ullmann's Encyclopedia of Industrial Chemistry, Vol. A14, 5^{th} Ed., Seite 399, letzter Absatz, und Seite 400) und kommerziell erhältlich.

Beispiele für im erfindungsgemäßen Verfahren einsetzbare makroporöse Kationenaustauscher in der H-Form sind:
Duolite® C264; Amberlite® 252, UP252 und 200; Imac® C16P; Lewatit® SP112 und K2621; Dowex® 88 und MSC1; Kastel® C300P; Ionac® CFP110; Relite® CFS und CFZ; Diaion® PK 220 und PK 228; Purolite C150, CT165 und CT175; Wofatit® KS10.
Bevorzugt werden Duolite® C264, Amberlite® 252 und Amberlite® UP252 eingesetzt.

Das erfindungsgemäße Verfahren findet Anwendung zur Reinigung von N-substituierten Lactamen der Formel I 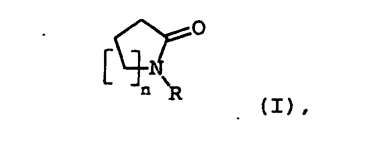 in der R einen
- linearen oder verzweigten gesättigten aliphatischen Rest, bevorzugt C₁₋₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, 2-Ethyl-hexyl, n-Nonyl, iso-Nonyl, n-Decyl, iso-Decyl, n-Undecyl, n-Dodecyl, iso-Dodecyl,
   besonders bevorzugt C₁₋₈-Alkyl, wie Methyl, Ethyl, n-Propyl, iso-Propyl, n-Butyl, iso-Butyl, sec.-Butyl, tert.-Butyl, n-Pentyl, und 2-Ethyl-hexyl,
   ganz besonders bevorzugt C₁₋₄-Alkyl, wie Methyl, Ethyl, n-Propyl, iso-Propyl, n-Butyl, iso-Butyl, sec.-Butyl, tert.-Butyl, und einen
- gesättigten cycloaliphatischen Rest mit 3 bis 12 C-Atomen, bevorzugt C₄₋₈-Cycloalkyl, wie Cyclobutyl, Cyclopentyl, Cyclohexyl, Cycloheptyl und Cyclooctyl, besonders bevorzugt Cyclopentyl und Cyclohexyl,
wobei in diesen Fällen der Rest R ein bis zwei unter den Reaktionsbedingungen inerte Hydroxy- oder C₁₋₈-Alkoxy-Substituenten tragen kann, wie z.B. R = C₁₋₄-Hydroxyalkyl oder C₁₋₈-Alkoxy substituiertes C₁₋₄-Alkyl,
und n eine ganze Zahl von 1 bis 4 bedeutet
und wobei der heterocyclische Ring des N-substituierten Lactams ein bis zwei Alkylreste, wie z.B. C₁₋₈-Alkylreste, die voneinander unabhängig sind, bevorzugt einen C₁₋₈-Alkylrest, tragen kann.

Als Substituenten in R kommen insbesondere in Betracht:
- Hydroxy und
- C₁₋₈-Alkoxy, wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy,
   besonders bevorzugt C₁₋₄-Alkoxy, wie Methoxy, Ethoxy, n-Propoxy, iso-Propoxy, n-Butoxy, iso-Butoxy, sec.-Butoxy und tert.-Butoxy.

Beispiele für C₁₋₈-Alkylreste, die der heterocyclische Ring des N-substituierten Lactams tragen kann, sind:
- Methyl, Ethyl, n-Propyl, iso-Propyl, n-Butyl, iso-Butyl, sec.-Butyl, tert.-Butyl, n-Pentyl, iso-Pentyl und 2-Ethyl-hexyl.

Das erfindungsgemäße Verfahren findet ganz besonders Anwendung zur Reinigung von N-substituierten Lactamen der der Formel I 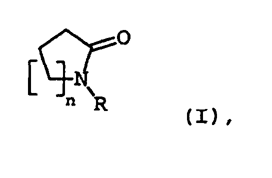 in der R C₁₋₄-Alkyl wie oben beschrieben bedeutet und einen Hydroxy-Substituenten tragen kann und n 1, 2 oder 3 bedeutet.

Beispiele für im erfindungsgemäßen Verfahren einsetzbare N-substituierte Lactame sind:

N-Methyl-2-pyrrolidon = N-Methyl-2-pyrrolidinon = N-Methyl-gamma-Butyrolactam (NMP), 1,3-Dimethyl-2-pyrrolidon, 1,4-Dimethyl-2-pyrrolidon, 1,5-Dimethyl-2-pyrrolidon, N-Ethyl-2-pyrrolidon, N-n-Propyl-2-pyrrolidon, N-iso-Propyl-2-pyrrolidon, N-n-Butyl-2-pyrrolidon, N-iso-Butyl-2-pyrrolidon, N-n-Pentyl-2-pyrrolidon, N-n-Hexyl-2-pyrrolidon, N-Cyclohexyl-2-pyrrolidon, N-Cyclopentyl-2-pyrrolidon, N-n-Decyl-2-pyrrolidon, N-n-Dodecyl-2-pyrrolidon, N-Benzyl-2-pyrrolidon, N-(2-Hydroxyethyl)-2-pyrrolidon, N-(3-Hydroxypropyl)-2-pyrrolidon, N-(2-Hydroxypropyl)-2-pyrrolidon, N-(2-Methoxyethyl)-2-pyrrolidon, N-(1-Methoxyethyl)-2-pyrrolidon, N-(2-Ethoxyethyl)-2-pyrrolidon, N-Methyl-delta-valerolactam, N-(2-Hydroxyethyl)-delta-valerolactam, N-Methyl-epsilon-caprolactam, N-Ethyl-epsilon-caprolactam, N-(2-Hydroxyethyl)-epsilon-caprolactam, N-Methyl-heptansäure-omega-lactam, N-(2-Hydroxyethyl)-heptansäure-omega-läctam, sowie Mischungen hiervon, bevorzugt NMP und N-(2-Hydroxyethyl)-2-pyrrolidon, besonders bevorzugt NMP.

Die im erfindungsgemäßen Verfahren eingesetzten N-substituierten Lactame werden bevorzugt mit einer Reinheit von größer 90 Gew.-%, insbesondere von größer 95 Gew.-%, z.B. als destillierte Ware, eingesetzt und können nach bekannten Verfahren, beispielsweise durch Umsetzung von Lactonen der Formel II 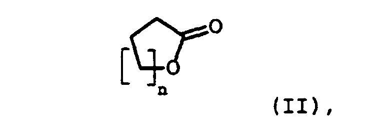 in der n eine ganze Zahl von 1 bis 4 bedeutet und wobei der heterocyclische Ring des Lactons ein bis zwei unter den Reaktionsbedingungen inerte Substituenten, wie z.B. C₁₋₈-Alkylreste, die von einander unabhängig sind, tragen kann, mit Aminen der Formel RNH₂, in der R die obengenannte Bedeutung hat, bei erhöhter Temperatur und erhöhtem Druck unter Freisetzung von einem Moläquivalent Wasser, z.B. gemäß Ullmann's Encyclopedia of Industrial Chemistry, Vol. A22, 5^{th} Ed., Seite 459 (1993), JP-A-74020 586 (Derwent Abstr. 46186V/25, JP-A-49000 259 (Derwent Abstr.21795V/12), DE-A-19 626 123, hergestellt werden.

N-substituierte 2-Pyrrolidone können auch durch Umsetzung von Maleinsäureanhydrid mit Aminen der Formel RNH₂ in Gegenwart von Wasserstoff und einem Katalysator hergestellt werden, z. B. gemäß EP-A-745 589.

Beispiele für Lactone der Formel II sind:
gamma-Butyrolacton, delta-Valerolacton, epsilon-Caprolacton, Heptansäure-omega-lacton, alpha-Methyl-gamma-butyrolacton, gamma-Valerolacton, gamma-Caprolacton, insbesondere gamma-Butyrolacton.

Beispiele für Amine der Formel RNH₂ sind:
Monomethylamin (MMA), Ethylamin, n-Propylamin, iso-Propylamin, n-Butylamin, Cyclopentylamin, Cyclohexylamin, n-Decylamin, n-Dodecylamin und Monoethanolamin, insbesondere MMA.

Das erfindungsgemäße Verfahren lässt sich wie folgt ausführen:

Die Behandlung des N-substituierten Lactams mit dem makroporösen Kationenaustauscher zur Reinigung des Lactams lässt sich diskontinuierlich, indem man beispielsweise in einem Kessel das Lactam in Gegenwart des Kationenaustauschers rührt, oder bevorzugt kontinuierlich, indem man beispielsweise das Lactam durch ein Rohr, in dem der Ionenaustauscher als Festbett angeordnet ist, fährt, durchführen. Geeignete Beispiele für Ausführungen und Rohre mit einem Ionenaustauscherfestbett finden sich z.B. in Ullmann's Encyclopedia of Industrial Chemistry, Vol. A14, 5^{th} Ed., Seite 431f, Kapitel 10.2 (1989).

Bei der kontinuierlichen Fahrweise mit einem in einem Rohr als Festbett angeordneten makroporösen Kationenaustauscher, wobei das Ionenaustauscherbett von unten nach oben (Sumpffahrweise) oder von oben nach unten durchströmt werden kann, liegt die spezifische Ionenaustauscherbelastung im allgemeinen bei 0,05 bis 100, insbesondere bei 0,1 bis 20, ganz besonders bei 0,1 bis 10 Bettvolumen pro Stunde (BV/h) und die sogenannnte Leerrohrgeschwindigkeit, d.h. der Volumenstrom ([m³/h]) pro Querschnittsfläche ([m²]) des Rohrs, im allgemeinen bei 0,5 bis 40, insbesondere bei 0,5 bis 20, ganz besonders bei 0,5 bis 10 m/h.

Man kann das Reinigungsverfahren auch in Gegenwart von inerten, polaren oder unpolaren, protischen oder aprotischen, Lösungsmitteln oder Mischungen hiervon, die mit dem Kationenaustauscher chemisch nicht reagieren, beispielsweise Wasser, Alkohole, wie Methanol, Ethanol, Isopropanol, Ether, wie Tetrahydrofuran, aromatische oder aliphatische Kohlenwasserstoffe, wie Toluol, Pentan oder Hexan, Dimethylsulfoxid (DMSO), durchführen, eine Arbeitsweise, die besonders dann in Betracht kommt, wenn das gereinigte Verfahrensprodukt in Form von entsprechenden Lösungen weiterverarbeitet wird.

Die Temperatur liegt im allgemeinen im Bereich von 5 bis 130°C, bevorzugt bei 10 bis 50°C. Besonders bevorzugt wird das erfindungsgemäße Verfahren bei Temperaturen von 15 bis 30°C durchgeführt.

Je nach Art des zu reinigenden N-substituierten Lactams bzw. dessen Lösung in einem Lösungsmittel wird die Temperatur mindestens so hoch eingestellt, dass das Reinigungsverfahren in flüssiger Phase abläuft.

Die Regenerierung der eingesetzten Kationenaustauscher erfolgt nach bekannten Methoden und Verfahren nach dem Gleichstrom- oder Gegenstromprinzip durch Behandlung mit einer wässrigen Lösung einer Brönsted-Säure, z. B. durch Behandlung mit 4 bis 10 Gew.-%iger, insbesondere ca. 7 Gew.-%iger, wässriger Chlorwasserstofflösung oder bevorzugt mit 0,5 bis 8 Gew.-%iger, insbesondere ca. 5 Gew.-%iger, wässriger Schwefelsäure.

Aufgrund der möglichen hohen Belastung (Bettvolumen) und der hohen Kapazität der im erfindungsgemäßen Verfahren verwendeten makroporösen Kationenaustauscher ergeben sich hohe Standzeiten dieser Ionenaustauscher und große Intervalle zwischen den Regenerierungsschritten. Im Besonderen kann der Austausch des beladenen Kationenaustauschers gegen frischen unbeladenen Kationenaustauscher wirtschaftlicher sein, als eine Regenerierungsstufe.

Im Übrigen unterliegt das erfindungsgemäße Verfahren keinen verfahrenstechnischen Besonderheiten, so dass sich weitere Angaben hierzu erübrigen.

Durch die erfindungsgemäße Behandlung der N-substituierten Lactame, insbesondere NMP, lassen sich Amine, insbesondere Amine der Formel RNH₂, ganz besonders Monomethylamin (MMA), die im allgemeinen in einer Konzentration von bis zu 10000 ppm, insbesondere 1000 ppm, ganz besonders 100 ppm, aber auch in Konzentrationen von größer 10000 ppm, als Verunreinigung enthalten sind, auf Konzentrationen kleiner 10 ppm, insbesondere kleiner 5 ppm, absenken, und zwar bei einer Belastung des makroporösen Kationenaustauschers von bis zu 200 Bettvolumen (BV), insbesondere von bis zu 500 BV, ganz besonders von bis zu 1000 BV (BV bezogen auf das lösungsmittelfreie N-substituierte Lactam).

Gleichzeitig lassen sich durch die erfindungsgemäße Behandlung der N-substituierten Lactame, insbesondere NMP, Metallkationen, wie Na⁺, K⁺, Ca²⁺, Mg²⁺, Fe²⁺ und Fe³⁺, insbesondere Na⁺, die im allgemeinen in einer Konzentration von bis zu 1000 ppb, insbesondere 100 ppb, ganz besonders 50 ppb, aber auch in Konzentrationen von größer 1000 ppb, als Verunreinigung vorliegen können, auf Konzentrationen kleiner 10 ppb, insbesondere kleiner 5 ppb, absenken, und zwar bei einer Belastung des makroporösen Kationenaustauschers von bis zu 200 Bettvolumen (BV), insbesondere von bis zu 500 BV, ganz besonders von bis zu 1000 BV (BV bezogen auf das lösungsmittelfreie N-substituierte Lactam).

Die ppm- und ppb-Angaben beziehen sich auf Gewichtsteile.

### Beispiele

### Vergleichsbeispiel

N-Methyl-2-pyrrolidon (NMP) mit einer Reinheit nach GC von 99,8 Fl.%, einem Natriumionengehalt von 30 ppb und einem Gehalt an Monomethylamin (MMA) von 52 ppm wurde in Sumpffahrweise bei 25°C durch ein Quarzglasrohr (Innendurchmesser: 30 mm, Länge: 100 cm), das mit dem stark sauren Kationenaustauscher Dowex® 650 C (Dow Chemical Company) vom Gel-Typ in der H-Form gefüllt war, gefahren. Das Bettvolumen (BV) des Ionenaustauschers betrug 620 ml. Die Leerrohrgeschwindigkeit betrug 1 m/h. Bei einem Volumenstrom von 1 BV/h (= spezifische Belastung) betrug der Gehalt an MMA im Ablauf des Glasrohrs bereits nach 50 zugeführten BV mehr als 5 ppm. Nach ca. 120 zugeführten BV betrug der Gehalt an MMA mehr als 10 ppm und nach 170 zugeführten BV mehr als 35 ppm.
Nach 170 zugeführten BV wurde eine Beladung des Ionenaustauschers mit nur 0,22 Mol MMA pro Liter Ionenaustauscher erreicht.

### Beispiel 1

Der Versuch wurde wie im Vergleichsbeispiel beschrieben durchgeführt, mit dem Unterschied, dass das Rohr mit dem stark sauren Kationenaustauscher Duolite® C 264 (Rohm und Haas Company) vom makroporösen Typ in der H-Form gefüllt war. Das Bettvolumen (BV) des Ionenaustauschers betrug 630 ml. Die Leerrohrgeschwindigkeit betrug 1 m/h.
Bei einem Volumenstrom von 1 BV/h (= spezifische Belastung) betrug der Gehalt an MMA im Ablauf des Glasrohrs nach 1000 zugeführten BV immer noch weniger als 5 ppm.
Nach 1000 zugeführten BV wurde eine Beladung des Ionenaustauschers mit 1,6 Mol MMA pro Liter Ionenaustauscher erreicht. Weiterhin betrug bei einem Volumenstrom von 1 BV/h der Gehalt Natriumionen im Austrag nach 1000 zugeführten BV ebenfalls immer noch weniger als 5 ppb.

### Beispiel 2

Der Versuch wurde wie im Vergleichsbeispiel beschrieben durchgeführt, mit dem Unterschied, dass das Rohr mit dem stark sauren Kationenaustauscher Amberlite® UP 252 (Rohm und Haas Company) vom makroporösen Typ in der H-Form gefüllt war. Das Bettvolumen (BV) des Ionenaustauschers betrug 630 ml. Die Leerrohrgeschwindigkeit betrug 1 m/h.
Bei einem Volumenstrom von 1 BV/h (= spezifische Belastung) betrug der Gehalt an MMA im Ablauf des Glasrohrs nach 1000 zugeführten BV immer noch weniger als 5 ppm.
Nach 1000 zugeführten BV wurde eine Beladung des Ionenaustauschers mit ca. 1,4 Mol MMA pro Liter Ionenaustauscher erreicht. Weiterhin betrug bei einem Volumenstrom von 1 BV/h der Gehalt Natriumionen im Austrag nach 1000 zugeführten BV ebenfalls immer noch weniger als 5 ppb.

## Patentansprüche

1. Verfahren zur Reduzierung des Amingehaltes von Amin-verunreinigten N-substituierten Lactamen, **dadurch gekennzeichnet, dass** man die so verunreinigten N-substituierten Lactame der Formel I 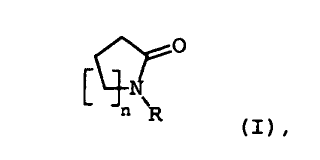 in der R einen linearen oder verzweigten, gesättigten aliphatischen oder cycloaliphatischen Rest darstellt, der ein bis zwei Substituenten, ausgewählt aus Hydroxy und C₁₋₈-Alkoxy, tragen kann, n eine ganze Zahl von 1 bis 4 bedeutet und wobei der heterocyclische Ring des N-substituierten Lactams ein bis zwei Alkylreste tragen kann, mit einem sauren makroporösen Kationenaustauscher behandelt.

2. Verfahren nach Anspruch 1, **dadurch gekennzeichnet, dass** die Behandlung mit einem stark sauren makroporösen Kationenaustauscher erfolgt.

3. Verfahren nach den Ansprüchen 1 oder 2, **dadurch gekennzeichnet, dass** der Kationenaustauscher ein Styrol-Divinylbenzol-Copolymer als Matrix aufweist.

4. Verfahren nach einem der Ansprüche 1 bis 3, **dadurch gekennzeichnet, dass** der Porendurchmesser des sauren makroporösen Kationenaustauschers 10 bis 150 nm beträgt.

5. Verfahren nach einem der Ansprüche 1 bis 4, **dadurch gekennzeichnet, dass** die nutzbare Kapazität des sauren makroporösen Kationenaustauschers mindestens 0,5 Mol Amin pro Liter Kationenaustauscher beträgt.

6. Verfahren nach einem der Ansprüche 1 bis 5 zur Reinigung von N-substituierten Lactamen der Formel I, in der R für C₁₋₄-Alkyl oder -Hydroxyalkyl steht, und n 1, 2 oder 3 bedeutet.

7. Verfahren nach einem der Ansprüche 1 bis 6 zur Reinigung von N-Methyl-2-pyrrolidon.

8. Verfahren nach einem der Ansprüche 1 bis 7, **dadurch gekennzeichnet, dass** man die N-substituierten Lactame bei Temperaturen von 5 bis 130°C mit dem Kationenaustauscher behandelt.

9. Verfahren nach einem der Ansprüche 1 bis 8, **dadurch gekennzeichnet, dass** man den Kationenaustauscher als Festbett anordnet.

10. Verfahren nach einem der Ansprüche 1 bis 9, **dadurch gekennzeichnet, dass** man N-substituierte Lactame einsetzt, die durch die Umsetzung von entsprechenden Lactonen mit primären Aminen erhalten wurden.

## Claims

1. A process for reducing the amine content of amine-contaminated N-substituted lactams, which comprises treating the contaminated N-substituted lactams of the formula I 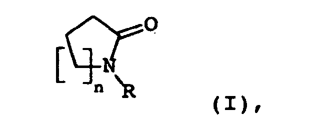 where R is a linear or branched, saturated aliphatic or cycloaliphatic radical which may bear one or two substituents selected from among hydroxy and C₁₋₈-alkoxy, n is an integer from 1 to 4 and the heterocyclic ring of the N-substituted lactam may bear one or two alkyl radicals, with an acid macroporous cation exchanger.

2. A process as claimed in claim 1, wherein the treatment is carried out using a strong acid macroporous cation exchanger.

3. A process as claimed in claim 1 or 2, wherein the cation exchanger has a styrene-divinylbenzene copolymer matrix.

4. A process as claimed in any of claims 1 to 3, wherein the pore diameter of the acid macroporous cation exchanger is from 10 to 150 nm.

5. A process as claimed in any of claims 1 to 4, wherein the utilizable capacity of the acid macroporous cation exchanger is at least 0.5 mol of amine per liter of cation exchanger.

6. A process as claimed in any of claims 1 to 5 for purifying N-substituted lactams of the formula I in which R is C₁₋₄-alkyl or hydroxyalkyl and n is 1, 2 or 3.

7. A process as claimed in any of claims 1 to 6 for purifying N-methyl-2-pyrrolidone.

8. A process as claimed in any of claims 1 to 7, wherein the N-substituted lactams are treated with the cation exchanger at from 5 to 130°C.

9. A process as claimed in any of claims 1 to 8, wherein the cation exchanger is employed as a fixed bed.

10. A process as claimed in any of claims 1 to 9, wherein the N-substituted lactams used are ones which have been obtained by reaction of the corresponding lactones with primary amines.

## Revendications

1. Procédé pour réduire le contenu en amines de lactames N-substitués contaminés par des amines, **caractérisé en ce qu'**on traite avec un échangeur de cations macroporeux acide les lactames N-substitués ainsi contaminés de formule I 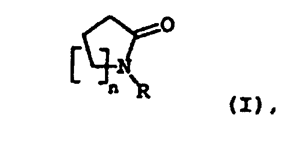 dans laquelle R représente un résidu cycloaliphatique ou aliphatique saturé, linéaire ou ramifié, résidu qui peut porter un à deux substituants choisis dans le groupe comprenant l'hydroxy et l'alkoxy en C₁ à C₈, n étant un nombre entier compris entre 1 et 4, et dans laquelle l'anneau hétérocyclique du lactame N-substitué peut porter un à deux résidus alkyles.

2. Procédé selon la revendication 1, **caractérisé en ce que** le traitement avec un échangeur de cations macroporeux fortement acide a lieu.

3. Procédé selon la revendication 1 ou 2, **caractérisé en ce que** l'échangeur de cations comprend un copolymère styrène-divinylbenzène comme matrice.

4. Procédé selon l'une quelconque des revendications 1 à 3, **caractérisé en ce que** le diamètre des pores de l'échangeur de cations macroporeux acide vaut de 10 à 150 nm.

5. Procédé selon l'une quelconque des revendications 1 à 4, **caractérisé en ce que** la capacité utile de l'échangeur de cations macroporeux acide vaut au moins 0,5 mol d'amine par litre d'échangeur de cations.

6. Procédé selon l'une quelconque des revendications 1 à 5 pour purifier des lactames N-substitués de formule I, formule dans laquelle R représente un alkyle en C₁ à C₄ ou un hydroxyalkyle et n vaut 1, 2 ou 3.

7. Procédé selon l'une quelconque des revendications 1 à 6 pour purifier la N-méthyl-2-pyrrolidone.

8. Procédé selon l'une quelconque des revendications 1 à 7, **caractérisé en ce qu'**on traite les lactames N-substitués avec l'échangeur de cations à des températures allant de 5 à 130°C.

9. Procédé selon l'une quelconque des revendications 1 à 8, **caractérisé en ce qu'**on dispose l'échangeur de cations comme lit fixe.

10. Procédé selon l'une quelconque des revendications 1 à 9, **caractérisé en ce qu'**on met en jeu des lactames N-substitués qui ont été obtenus par la transformation des lactones correspondantes avec des amines primaires.
